(19) Europäisches Patentamt European Patent Office Office européen des brevets

(11) **EP 3 899 925 B1**

(12) **EUROPEAN PATENT SPECIFICATION**

(45) Date of publication and mention
of the grant of the patent:
**14.06.2023 Bulletin 2023/24**

(21) Application number: **19816741.3**

(22) Date of filing: **12.12.2019**

(51) International Patent Classification (IPC):
**G10K 11/175** (2006.01)     **A61B 5/00** (2006.01)
**A61B 5/369** (2021.01)     **A61M 21/00** (2006.01)
**A61M 21/02** (2006.01)

(52) Cooperative Patent Classification (CPC):
(C-Sets available)
**A61B 5/4806; A61B 5/369; A61M 21/02;**
**G10K 11/1752;** A61M 2021/0027;
A61M 2205/3375; A61M 2205/3592; A61M 2205/50;
A61M 2205/70; A61M 2230/10          (Cont.)

(86) International application number:
**PCT/EP2019/084782**

(87) International publication number:
**WO 2020/126777 (25.06.2020 Gazette 2020/26)**

(54) **A NOISE MASKING DEVICE AND A METHOD FOR MASKING NOISE**

LÄRMMASKIERUNGSVORRICHTUNG UND VERFAHREN ZUR MASKIERUNG VON LÄRM

DISPOSITIF DE MASQUAGE DE BRUIT ET PROCÉDÉ POUR MASQUER LE BRUIT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.12.2018 EP 18212981**

(43) Date of publication of application:
**27.10.2021 Bulletin 2021/43**

(73) Proprietor: **Koninklijke Philips N.V.**
**5656 AG Eindhoven (NL)**

(72) Inventors:
• **VAN DEN ENDE, Daan Anton**
**5656 AE Eindhoven (NL)**
• **PASTOOR, Sander Theodoor**
**5656 AE Eindhoven (NL)**

(74) Representative: **Philips Intellectual Property &**
**Standards**
**High Tech Campus 52**
**5656 AG Eindhoven (NL)**

(56) References cited:
**EP-A1- 1 886 707          WO-A1-2018/053114**
**WO-A1-2018/166625**

(52) Cooperative Patent Classification (CPC): (Cont.)

C-Sets
A61M 2230/10, A61M 2230/005

**Description**

FIELD OF THE INVENTION

The present invention relates to the field of systems for mitigating unwanted acoustic noise, and in particular to the field of noise masking systems.

BACKGROUND OF THE INVENTION

Unwanted acoustic noise can disturb people or subjects. The disturbance to a person can be mitigated by playing a masking or anti-noise (i.e. noise-cancelling) sound. Such sounds can be generated and played through an external device (e.g. speakers or a smartphone), or can be generated and played by hardware forming part of a noise masking system.

A masking sound is typically a recorded repetitive sound (such as rain or ocean waves) or a generated random waveform with equally distributed acoustic intensity over the audible frequency range (termed 'white noise'). These sounds all aim to drown out sudden and/or annoying external noise and can be clustered under the term 'masking sound'.

In particular, a masking sound can mask acoustic noise that would otherwise disturb a user, e.g. during sleep.

Anti-noise (sound cancellation) is a special form of masking sound which needs a microphone close to the ear to pick up the sound vibrations in order to play the right phase-shifted anti-noise.

A masking sound volume should be high enough to drown out the unwanted noise. However, noise levels may change, which would necessitate a corresponding different volume for the masking sound. Typical masking sound generators operate without feedback. Thus, the user must manually balance the volume settings to drown out the unwanted noise while also avoiding disturbance from an overly loud playback of the masking sound itself.

Some masking sound generators implement an adaptive function that adjusts the masking sound volume as a function of the background (room) noise volume. This enables automatic volume adjustment of the masking sound based directly on the room noise level.

EP 1 886 707 discloses a device for monitoring noises in a sleeping environment and for generating a calming or soothing sound output to mask the noise and encourage sleep. The device detects ambient noise in a sleeping environment and also monitors the user's vital signs to derive the user's sleep state. Based on the determined sleep state, the device is adapted to control the characteristics of the audio output, such as tempo and volume.

WO2018053114 discloses a sleep assistance device including a contactless biometric sensor, a processor, memory, and a speaker. The processor detects a user's sleep state by reading signals from the contactless biometric sensor. The processor also plays noise-masking sounds upon detecting that a user has fallen asleep.

WO2018166625 discloses an apparatus for enabling adaptive audio signal alteration. When an input audio signal is received, a determination of whether the user of an audio device hears the input audio signal is performed based upon brain activity of the user. A determination of whether the user is distracted by the audio signal is performed based upon sensor measurements indicating a physical state of the user. In response to determining that the user hears the input audio signal and that the input audio signal causes the user to be distracted, a determination of configuration parameter(s) is performed. An alteration of audio signal(s) is caused based upon the configuration parameter(s) to obtain modified version(s) of the audio signal(s) that are intended to address the distraction caused by the input audio signal, and output audio signals are output, where the output audio signals include the modified versions.

However, a problem with controlling the volume of masking sounds is that the masking sounds themselves may then become a source of disturbance. This may depend on the sensitivity of the user to the masking sounds. There is a desire to optimize the operation of masking sound generators, and in particular to provide suitable masking sounds that minimize or reduce a disturbance to a user.

SUMMARY OF THE INVENTION

The invention is defined by the claims.

According to examples in accordance with an aspect of the invention, there is provided a device for masking noise, the device comprising:

a transducer unit for detecting noise to be masked;
a sound generating unit for generating a masking sound;
a sensor unit for monitoring a user's brain activity; and
a controller adapted to:

during a calibration, determine the user's brain activity, measured by the sensor unit, in response to a calibration sound, generated by the sound generating unit; and
during use of the device, adjust signal characteristics of the masking sound based on:

the noise detected by the transducer unit;
the user's brain activity in response to the noise detected by the transducer unit; and
the user's brain activity in response to the calibration sound generated by the sound generating unit during the calibration.

The device generates a masking sound to mask external noise, e.g. for use during sleep. In examples, the external noise to be masked is one that would otherwise disturb the user's sleep. The device automatically adjusts the signal characteristics of the masking sound based on the noise level in the environment, the user's response to this environmental noise level and the user's sensitivity to the masking sound (as previously determined during a calibration). Thus, the masking sound is able to mask the external noise whilst taking the user's sensitivity to the masking sound into account. This could, for example, mitigate unintentional disturbances to the user (or the user's sleep) due to an overly loud playback of the masking sound itself or disturbances caused by a user's sensitivity to a certain frequency or frequencies of acoustic noise. The sensitivity may be determined during the calibration stage as discussed in more detail below. The method of adjusting the signal characteristics of the masking sound based on the noise detected by the transducer unit is for example particularly suited to masking frequently occurring noises (for example traffic or snoring).

The signal characteristics of the masking sound may comprise at least one of a signal volume and a signal frequency. Thus, the device is able to adjust the loudness, the pitch, the quality, the type, and/or the tone of the masking sound in order to optimally mask the external noise. This function is well suited for noises which occur frequently, recurrently (but unpredictably), with a known frequency, and for a time period longer than a few seconds.

The sound generating unit may be adapted, during the calibration, to generate a calibration sound and the controller is adapted to determine the user's brain activity in response to the calibration sound, set upper and lower volume limits for the masking sound based on the user's brain activity in response to the calibration sound.

These features provide the method of an automatic device calibration that enables the device to set limits for the volume of the masking sound based on the user's hearing sensitivity.

In the context of the present application, the term "during use of the device" is used to mean whilst the device is outputting or generating an audible masking sound, i.e. after the masking sound is generated.

The calibration may take place before use of the device to generate the audible masking sound, e.g. in an initial set-up process. In particular examples, the device is adapted so that no calibration can take place during use of the device (i.e. whilst the device is operating so as to mask a noise detected at the transducer unit).

The user's brain activity, measured by the sensor unit, may comprise an electroencephalography (EEG) response. Thus, the sensor unit may comprise an electroencephalography system, e.g. formed of one or more electrodes. Measurement of the EEG response comprises measurement of the auditory steady-state response (ASSR), auditory brainstem response (ABR) or event related potentials (ERPs). These measurements correlate to the sensitivity of the human ear. This information can therefore be used for accurate calibration of the signal characteristics (e.g. volume) of the masking sound.

The device of the invention comprises a memory storage unit, in communication with the controller, adapted to store noise data based on the noise detected by the transducer unit. The controller is adapted to analyze the noise data using an algorithm, determine an expected noise trend, and adjust the signal characteristics of the masking sound based on the expected noise trend. Thus, the device is able to store and analyze the trend of repetitive noises over typical nights, enabling the device to predict the noise level expected to occur in the environment. Predictions can be based on metadata, such as day of the week, time of the year, and previous sleep cycles.

In a further embodiment, the algorithm used to analyze the noise data may be a machine-learning algorithm. The machine-learning algorithm enables the device to improve the prediction of the expected noise trend based on previously recorded data.

The device may further comprise a user-interface unit, in communication with the controller, adapted to enable the user to manually adjust the volume of the masking sound. This enables the user to interact and control features of the device, such as adjusting the volume of the masking sound and performing a manual device calibration.

The sound generating unit may be adapted, during the calibration, to generate a calibration sound, the user-interface unit is adapted to receive user feedback on the calibration sound, and the controller is adapted to set an upper volume limit and a lower volume limit for the masking sound based on the user feedback.

This provides a manual device calibration that enables the user to provide feedback using the user-interface unit, enabling the system to adapt the sound intensity from the sound generating unit to the level of the ASSR, so that differences in audio delivery and reception between nights can be compensated. This calibration can be implemented in conjunction with the automatic device calibration as a supplementary calibration, in order to further improve the accuracy of the device's ability to mask noise.

In a further embodiment, the sensor unit may be further adapted to detect a user's sleep state based on the user's measured brain activity. The controller may be further adapted to adjust the signal characteristics of the masking sound based on the user's sleep state. Thus, the device automatically adjusts the signal characteristics of the masking sound based on the user's sleep state and optionally the timing of transitions between different sleep states.

In a further embodiment, the controller may be further adapted to determine a recommended adjustment of the signal characteristics of the masking sound based on the user's sleep state. The user-interface unit may be further adapted to notify the user of the recommended signal characteristic adjustment. Thus, the device recommends to the user whether the signal characteristic should be adjusted based on the user's sleep state, enabling the user to manually adjust this setting. In particular, the signal characteristics may be adjusted when the system has detected arousals or awakenings (detected based on the determined sleep state).

This procedure may for example involve recommending volume adjustment to the user based on user data relating to the user's sleep pattern collected in previous nights, and in particular relating to detected awakenings, arousals, or regularity of light and deep sleep.

In a preferred embodiment, the sensor unit may be in wireless communication with the controller. This enables the sensor unit to be physically detached from the rest of the device, improving the user comfort should the sensor unit be a wearable component.

The masking sound generated by the sound generating unit may be a continuous calming sound adapted to relax the user. The continuous calming sound improves the user's sleep quality by relaxing the user and reducing stress.

The invention also provides a method for masking a noise, the method comprising:

during a calibration, determining the user's brain activity in response to a calibration sound;
detecting a noise to be masked;
monitoring a user's brain activity; and
generating a masking sound, and adjusting the signal characteristics of the masking sound based on the detected noise, the user's brain activity in response to the noise, and the user's brain activity in response to the calibration sound.

This method provides a masking sound adapted to mask external noise for example for use during sleep. For example the external noise that is masked could otherwise disturb the user's sleep.

The method of calibrating the device may comprise:

generating a calibration sound; and
monitoring the user's brain activity in response to the calibration sound, and
wherein adjusting the signal characteristics of the masking sound comprises:
setting upper and lower volume limits for the masking sound based on the user's brain activity in response to the calibration sound.

This method provides an automatic device calibration that enables setting limits for the volume of the masking sound based on the user's hearing sensitivity.

In a further or alternative embodiment, the method of calibrating the device may comprise:

generating a calibration sound;
receiving user feedback on the calibration sound; and
setting an upper volume limit and a lower volume limit for the masking sound based on the feedback provided by the user.

This method provides a manual device calibration. The user may provide feedback on the calibration sound, which enables control of the sound intensity of the calibration sound based on the feedback. This method of calibration may be used as a supplementary calibration method in addition to the automatic calibration method, to further improve the accuracy of the noise masking.

The method of the invention comprises storing noise data based on the detected noise to be masked; analyzing the noise data using an algorithm, preferably wherein the algorithm is a machine-learning algorithm; determining an expected noise trend; and adjusting the signal characteristics of the masking sound based on the expected noise trend.

There is also proposed a computer program comprising code means for implementing any previously described method when said program is run on a computer.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a noise masking device according to an embodiment;
Fig. 2 shows a use-case scenario of the noise masking device in the presence of unwanted acoustic noise;
Fig. 3 shows a first method for calibrating the noise masking device according to an embodiment;
Fig. 4 shows a second method for calibrating the noise masking device according to an embodiment; and
Fig. 5 shows a method for adjusting the signal characteristics of a generated masking sound generated using the noise masking device.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device (and method) for masking noise in which a calibration is carried out to determine the sensitivity of a user to a calibration sound. During use of the device, the signal characteristics of the masking sound are adjusted based on the detected noise, the response of the user to the detected noise and also the response of the user to the calibration sound. As a result, a masking sound is generated that is optimally adapted to mask unwanted noise, in particular in a way which avoids the masking noise itself becoming a disturbance to the particular user.

Embodiments are at least partly based on the realization that, although masking sounds are adapted to mask external noise that may otherwise disturb a user, there exists a risk that the masking sound itself may contribute to the user's disturbance. It has been recognized that it is possible to regulate the masking sound based on the user's sensitivity to the masking sound.

Illustrative embodiments may, for example, be employed to improve a user's sleep quality by reducing the disturbance caused by external noises during a user's sleep.

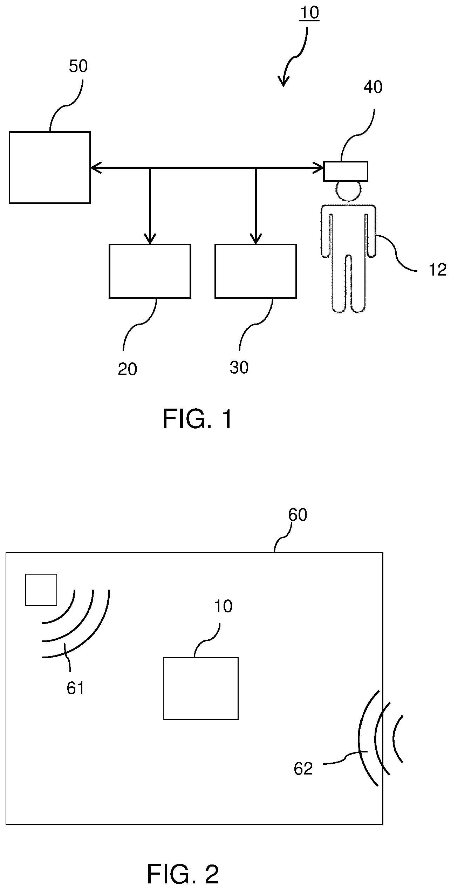

Fig. 1 shows a noise masking device 10, comprising a transducer unit 20 for detecting sound, a sound generating unit 30, a sensor unit 40 for detecting brain activity of the user 12, and a controller 50. The controller 50 is adapted to mask a detected noise using a generated masking sound.

The transducer unit 20 is adapted to detect sound, for example unwanted acoustic noise in an environment of a user 12. The transducer unit 20 may comprise a microphone.

The sound generating unit 30 is adapted to generate sound, for example a continuous tone or repeating tune. The generated sound is adapted to mask noise detected by the transducer unit 20 and can thereby be described as a "masking sound".

The sensor unit 40 is adapted to monitor the user's brain activity. Monitoring of brain activity provides a method of detecting a user's response to a particular sound. In a preferred example, the method of monitoring brain activity may be based on electroencephalography (EEG). Measuring an EEG response may include determining the auditory steady-state response (ASSR) or event related potentials (ERPs) of the user 12. A typical method of measuring an EEG response uses non-invasive electrodes placed along the user's scalp. When a user 12 hears a sound, a measurable EEG response is detected. In a preferred embodiment, the sensor unit 40 may include a wearable headset comprising non-invasive electrodes adapted to measure the user's EEG response.

The controller 50 is in communication with the transducer unit 20, the sound generating unit 30, and the sensor unit 40. In a preferred embodiment, the controller 50 is in wireless communication with at least the sensor unit 40 (and optionally other units as well, such as the sound generating unit). An operation of the controller 50 will be described below.

Fig. 2 shows an example use of the device 10. When in use, the device 10 may be located in an enclosed environment 60, such as a bedroom. Noise detected by the device may include noise 61 with an origin located inside the enclosed environment or noise 62 with an origin located outside the enclosed environment. The detected noise may further comprise a combination of the noise from these origins. Examples of sources of noise 61 from within an enclosed environment include electrical appliances, snoring partners, and insects. Examples of sources of noise 62 from outside an enclosed environment include traffic, aircraft, neighbors, construction work, and insects.

To operate the device, a calibration is first carried out. The purpose of the calibration is to determine the user's brain activity in response to a calibration sound, generated by the sound generating unit 30. This calibration sound is of the same type as will be generated to be a masking sound during subsequent use of the system. Thus, it enables a determination of how the user will respond to the masking sound, and thereby enables control of the masking sound to ensure that the masking sound itself does not become a source of disturbance to the user's sleep.

The calibration therefore takes place before use of the device to generate the audible masking sound, e.g. in an initial set-up process. In particular examples, the device is adapted so that no calibration can take place during use of the device (i.e. whilst the device is operating so as to mask a noise detected at the transducer unit).

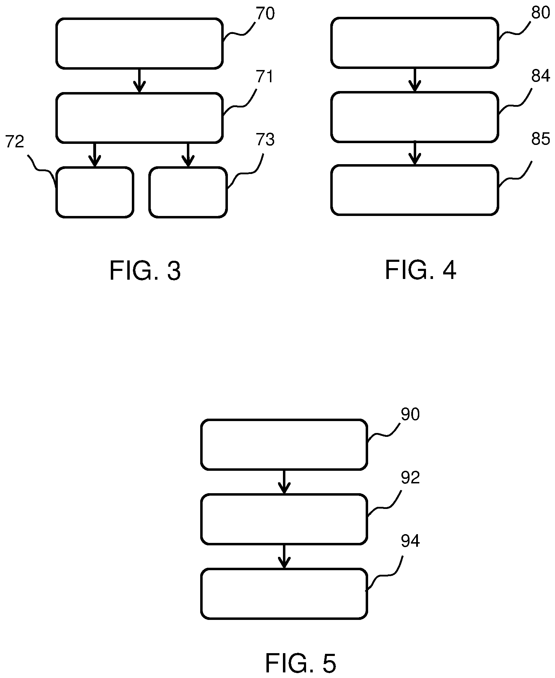

Fig. 3 shows a first possible calibration method, which provides automatic calibration of the device 10. In an initial step 70, the sound generating unit 30 produces a calibration sound. In a step 71, the controller 50 monitors the user's brain activity based on the calibration sound, enabling limits or bounds to be set for the volume of the masking sound. Sounds above the (user specific) threshold will generate a measurable ASSR or ABR, indicating the sensitivity of the user's hearing, i.e. a point at which the user 12 would begin to be disturbed by the volume of the masking sound.

In step 72, the upper limit for the masking sound may therefore be set based on the user's brain activity in response to the calibration sound, whilst in a step 73, the lower limit is determined based the point at which a measurable ASSR or ABR is detected.

For this purpose, the generation of the calibration sound involves sweeping characteristics of the calibration sound, in particular the volume but optionally also the frequency, frequency spectrum or other characteristics, in order to determine the user's response to different sound types. Thus, the characteristics of the user's hearing are determined, such as the volume and/or frequency range of their hearing capabilities.

As explained further below, the result of the calibration may be to derive a calibration constant. This may be set at a default value for the average user, or an average for the user's age group and/or gender. The calibration constant is then adjusted based on a determined ratio of the user's hearing threshold compared to the average hearing threshold. For example, an average hearing threshold for a normal man aged 20 is 3dB at 2kHz. If the user's threshold is determined at 10dB the calibration constant may be set 5 times as high as the default value to achieve a similar perceived noise level for this particular user.

Note that ASSR or ABR detection are only examples. It is also possible to measure the heart rate response to sound, as described in Roessler, R. , Collins, F. and Burch, N. R. (1969), HEART RATE RESPONSE TO SOUND AND LIGHT. Psychophysiology, 5: 359-369.

Fig. 4 shows a second possible calibration method. In step 80, the sound generating unit 30 produces a calibration sound. As discussed above, this involves a sweep of sound characteristics.

In a step 84, the device 10 requests the user's feedback based on the perceived sound level of the masking sound (e.g. low, moderate, high intensity sound) and the comfort level. This may be correlated with the ASSR or ABR response for a personal calibration of the device 10. In a step 85, the upper and lower limits of the masking sound volume may be set based on manual input feedback provided by the user 12 in response to the calibration sound. For example, if the user 12 selects a "low" option, this may correspond to the lower limit, and if the user 12 selects a "high" option, this may correspond to the upper limit. The device may comprise a user-interface unit to enable the user 12 to manually input response information.

The calibrated device 10 is then used to adapt the masking sound volume produced by the sound generating unit 30 to the level of the user's measured ASSR or other measure of hearing characteristics, so that differences in audio delivery and reception due to the user's hearing characteristics but also for changes in positioning of the sound generating unit 30 can be compensated.

Fig. 5 shows a method for masking noise using the device 10 of Fig. 1. This method follows one (or even both) of the calibration methods described above.

In an initial step 90, the transducer unit 20 detects a noise to be masked. In step 92, the sensor unit 40 measures the user's brain activity, which is thus in response to the detected noise.

These two steps enable the noise to be detected by the transducer unit and also enable monitoring of the user's brain activity in response to the noise detected by the transducer unit. Thus, it can be determined if noise masking is needed based on whether the user's brain activity shows that the user has been disturbed.

If noise masking is needed the sound generating unit 30 generates a masking sound in step 94. The masking sound takes account of the previous device calibration. Thus, the signal characteristics of the masking sound are adjusted based on the detected noise, the user's brain activity in response to the noise, and the user's brain activity in response to the calibration sound.

The signal characteristics of the masking sound which are adjusted for example comprise:

the volume;
the frequency (for a single tone);
the frequency spectrum (for a noise based signal), such as a type of noise;
the temporal characteristics such as a volume function over time.

The frequency spectrum may for example be adapted to match the hearing capabilities of the user. For example, a user will have a particular hearing response to different frequencies (as may be determined during the calibration) so that a white noise signal may be adapted so that instead of having a flat amplitude as a function of frequency, the amplitude follows the inverse of the hearing sensitivity of the user, so that the user perceives a white noise sound.

There may be frequencies to which the user is particularly sensitive (either their hearing or their brain response), and these may be suppressed in the masking sound.

Typical examples of noise detected during a period of sleep include traffic noise or snoring sounds. The adjusted signal characteristics typically include the signal volume and/or the signal frequency, although other options are outlined above.

The transducer unit 20 measures the frequency of the detected noise and the sensor unit 40 analyzes the user's EEG spectrum for determination of an ASSR or ABR. The frequency which may enable the detection of an ASSR peak as a result of the detected noise is known by the controller 50.

By way of example, the ASSR is an electrophysiological response to rapid auditory stimuli obtained by applying a carrier stimulus at a certain repetition rate, for instance every 10 milliseconds. Test frequencies used are commonly 500, 1000, 2000, and 4000 Hz. These frequencies are for example amplitude modulated. The brain signal that is recorded in the EEG spectrum is a response to the auditory carrier stimulus and is only present if the auditory carrier stimulus is registered by the ear (i.e. below the hearing threshold no response signal will be present in the EEG spectrum, above the threshold the response is visible in the spectrum).

If the detected noise is registered in the EEG spectrum, the masking sound volume is adjusted to the level of the noise. This function is well suited for sound which occurs frequently and recurrently (but unpredictably), with a known frequency (such as railroad, aircraft, traffic, and snoring sounds) and for a time period longer than a few seconds for each noise-producing event. This enables the masking sound to be optimally tuned to the detected noise, so that the noise masking is improved, with a lower risk of discomfort for the user 12 due to disturbance caused by the masking sound.

The volume of the masking sound is thus adjusted based both on the noise detected by the transducer unit 20 (so that the masking sound is able to mask the noise) and on the user's response (so that the masking sound does not disturb the user).

The user 12 may for example set the volume of the masking sound before going to sleep, such that the masking sound volume is sufficient to mask the unwanted noise at the time the user goes to sleep. If the noise level changes over time the masking sound volume is adjusted accordingly to ensure the unwanted noise remains masked by the masking sound.

The masking sound power set point (i.e. the power of the masking sound at a given time, where power corresponds to sound level) can be defined as:

$$P_{set} = P_{init} + C\left(P_{ext} - P_{ext\_init}\right) \qquad (1)$$

where $P_{set}$ is the masking sound power set point, $P_{init}$ is the initial masking sound power at the volume set by the user 12 at the start of the session, $P_{ext}$ is the sound power of the unwanted noise at a given time, $P_{ext\_init}$ is the initial sound power of the unwanted noise, and C is a calibration constant (e.g. having a default value C=1).

In this way, the masking sound power is initially set to match the unwanted noise, and this may be carried out by the user before they sleep. As the noise changes, the set point is adapted in response to changes in the noise power, taking account of the user's personal characteristics embodied in the calibration constant C.

As mentioned above, the calibration steps may for example involve setting the value of the calibration constant, C as well as setting a maximum for the value $P_{set}$.

The calibration constant may be set automatically or manually based on the user's hearing sensitivity determined during the calibration. With the calibration constant C set at a default level, the system outputs sounds which are audible for an average person. Mappings between calibration constants and hearing characteristics are stored in the system, for instance based on the sound level threshold at which an ASSR is detected. For users with less sensitive hearing, the calibration sequence will detect a larger audio level threshold at which an ASSR response is present and then will set the calibration constant C accordingly, for instance using a Decibel scale.

In a preferred embodiment, the trend of the repetitive noise (e.g. over typical nights) may be stored and analyzed. Based on metadata (such as the day of the week and the time of year) and/or personal data (such as previous sleep/wake times of the user 12), the set point ($P_{set}$) may be further adjusted to better predict the sound level of the detected noise that is expected to occur based on a (typical) reference sound level during such a night ($P_{ext\_ref}$ at time t versus the reference sound level of the unwanted noise at the start of the session (time t0)):

$$P_{set}(t) = P_{init} + C\left(P_{ext} - P_{ext\_init}\right)\left(\frac{P_{ext\_ref}(t)}{P_{ext\_ref}(t0)}\right) \qquad (2)$$

where $P_{ext\_ref}(t)$ is the reference sound power of the unwanted noise at a given time t, and $P_{ext\_ref}(t0)$ is the initial reference sound power at the start of the session.

As shown, the adjustment to the initial power is scaled based on the ratio between the noise level at the particular time and a noise level at a reference time t0.

The value P_ext can is measured at particular set points rather than continuously which means it will be lagging with respect to the external noise level. This is not an issue when the noise decreases over time, but it is a problem when there is an increase in noise during the night. Thus, a predictive element is incorporated which can anticipate the increase in sound ahead of time and ensure that the masking sound is adjusted for it.

This is for example of interest when a large but predictable increase in external noise level occurs during the night, for instance a train passing by or another person's alarm clock.

The noise-masking device of the invention includes a memory storage unit, which stores noise data based on the noise detected by the transducer unit 20. The memory storage unit may be in communication with the controller 50, which is adapted to analyze the noise data using an algorithm. This algorithm (for instance, a machine-learning algorithm) takes into account previous data to predict the trend of unwanted noise sound level. The controller 50 is further adapted to determine an expected noise trend based on the noise data analysis. This controller 50 thereafter adjusts the signal characteristics of the masking sound based on the expected noise trend.

The device 10 may include a user-interface unit adapted to enable the user 12 to manually adjust the volume of the masking sound. The user-interface unit may be in communication with the controller 50.

In a certain embodiment, the signal characteristics of the masking sound may be adjusted based on a user's sleep state detected by the sensor unit 40. The sensor unit 40 measures the user's brain activity and derives sleep relevant features such as awakenings, arousals, and regularity of light and deep sleep (e.g. number of exits out of deep sleep).

Based on the sleep relevant features derived from previous sleep sessions, the controller 50 may recommend the user 12 to change the volume or frequency of the masking sound, or automatically adapt the masking sound.

The recommended signal characteristics adjustment may be communicated to the user 12 through a notification displayed using the user-interface unit. In a preferred embodiment, the transducer unit 20 may measure and record data on noise levels during a user's sleeping period in order to produce data on expected noise levels by means of an algorithm. The controller 50 may be adapted to generate the recommended adjustment of the signal characteristics based on the produced data, and the user-interface unit may be adapted to provide the produced data and the ability to adjust the signal characteristics based on the data to the user 12.

The system may additionally be used to generate continuous calming sounds adapted to relax the user 12 when they are going to sleep. This is an additional feature to the masking of noise, and is known.

The calming sound is designed to calm the user 12 in order to ease the process of falling asleep. Thus, in addition to delivering masking sounds during sleep, the system may also be used to play calming sounds to initiate sleep. These calming sounds may also be noise based signals, or they may be other signals, such as speech, whale noises etc.

The sound generating unit 30 may also deliver auditory stimulation while a user 12 is sleeping to enhance sleep slow waves without causing arousals. This is an additional feature to the masking of noise, and is known. Such sounds for example comprise pulses of sound, for example with 50ms duration separated by 1 second pauses. This enables cognitive benefits and enhancement of sleep restoration. There are different causes that can prohibit falling asleep (e. g. before first sleep onset or falling back asleep after waking up later during the night). These are generally categorized in two sets, internal and external causes. External causes may include unwanted acoustic noise, as described above. Internal disturbances may include psychological causes (e.g. stress, rumination), physiological causes (e.g. low sleep pressure, tinnitus, hypertension), and behavioral causes (e.g. poor sleep hygiene). By playing a continuous calming sound during the user's sleep the device 10 may improve the user's sleep quality.

Thus, in addition to delivering masking sounds during sleep, the system may also be used to play sounds to promote deep sleep.

Applications of the invention may include, but are not limited to, any application which includes one or more of a sleep tracking system, a sound delivery system, and a sound level measurement system. For example a cardiorespiratory-based sleep tracker in combination with a smartphone or wake-up light.

The skilled person would be readily capable of developing a controller for carrying out a previously described method. Thus, each step of the flow chart may represent a different action performed by a controller, and may be performed by a respective module of the controller.

As discussed above, embodiments make use of a controller. The controller can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g. one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A device (10) for masking noise, the device (10) comprising:

   a transducer unit (20) for detecting noise to be masked;
   a sound generating unit (30) for generating a masking sound;
   a sensor unit (40) for monitoring a user's brain activity;
   a controller (50) adapted to:

      during a calibration, determine the user's brain activity, measured by the sensor unit (40), in response to a calibration sound, generated by the sound generating unit (30); and
      during use of the device (10), adjust signal characteristics of the masking sound based on:

         the noise detected by the transducer unit (20);
         the user's brain activity in response to the noise detected by the transducer unit (20); and
         the user's brain activity in response to the calibration sound generated by the sound generating unit (30) during the calibration;

   a memory storage unit, in communication with the controller (50), adapted to store noise data based on the noise detected by the transducer unit (20); and
   **characterized in that** the controller (50) is further adapted to:

      analyze the noise data using an algorithm, preferably wherein the algorithm is a machine-learning algorithm;
      determine an expected noise trend; and
      adjust the signal characteristics of the masking sound based on the expected noise trend.

2. A device (10) as claimed in claim 1, wherein the signal characteristics of the masking sound comprise at least one of:

   a signal volume; and
   a signal frequency.

3. A device as claimed in claim 2, wherein:

the sound generating unit (30) is adapted, during the calibration, to generate a calibration sound; and
the controller (50) is adapted to:

determine the user's brain activity in response to the calibration sound;
set an upper and lower volume limits for the masking sound based on the user's brain activity in response
to the calibration sound.

4. A device (10) as claimed in any of claims 1 to 3, wherein the sensor unit (40) comprises an electroencephalography (EEG) system, so that the user's brain activity measured by the sensor unit (40) comprises an electroencephalography response.

5. A device as claimed in any of claims 1 to 4, further comprising a user-interface unit, in communication with the controller (50), adapted to enable the user (12) to manually adjust the volume of the masking sound.

6. A device as claimed in claim 5, wherein:

the sound generating unit (30) is adapted, during the calibration, to generate a calibration sound;
the user-interface unit is adapted to receive user feedback on the calibration sound; and
the controller (50) is adapted to set an upper volume limit and a lower volume limit for the masking sound based
on the user feedback.

7. A device as claimed in any of claims 1 to 6, wherein:

the sensor unit (40) is further adapted to detect a user's sleep state; and
the controller (50) is further adapted to adjust the signal characteristics of the masking sound based on the
user's sleep state.

8. A device as claimed in claim 7, wherein:

the controller (50) is further adapted to determine a recommended adjustment of the signal characteristics of
the masking sound based on the user's sleep state; and
the user-interface unit is further adapted to notify the user (12) of the recommended adjustment.

9. A device as claimed in any of claims 1 to 8, wherein the sensor unit (40) is in wireless communication with the controller (50).

10. A method for masking noise, the method comprising:

(70,71,72,73; 80,84,85) during a calibration, determining the user's brain activity in response to a calibration
sound;
(90) detecting a noise to be masked;
(92) monitoring a user's brain activity; and
(94) generating a masking sound, and adjusting the signal characteristics of the masking sound based on the
detected noise, the user's brain activity in response to the noise, and the user's brain activity in response to the
calibration sound.
storing noise data based on the detected noise to be masked;
analyzing the noise data using an algorithm, preferably wherein the algorithm is a machine-learning algorithm;
determining an expected noise trend; and
adjusting the signal characteristics of the masking sound based on the expected noise trend.

11. A method as claimed in claim 10, wherein the calibration method comprises:

(70) generating a calibration sound; and
(71) monitoring the user's brain activity in response to the calibration sound, and
wherein adjusting the signal characteristics of the masking sound comprises:
(72,73) setting upper and lower volume limits for the masking sound based on the user's brain activity in response
to the calibration sound.

12. A method as claimed in any of claim 10 or 11, wherein the step of determining the user's brain activity in response to a calibration sound comprises:

(80) generating a calibration sound;
(84) receiving user feedback on the calibration sound; and
(85) setting upper and lower volume limits for the masking sound based on the user feedback.

13. A computer program comprising instructions which, when the program is executed by a computer, cause the computer to carry out the method of any one of claims 10 to 12.

**Patentansprüche**

1. Vorrichtung (10) zum Maskieren von Geräusch, wobei die Vorrichtung (10) umfasst:

Eine Wandlereinheit (20) zum Erfassen von zu maskierendem Geräusch; eine Tonerzeugungseinheit (30) zum Erzeugen eines maskierenden Tons; eine Sensoreinheit (40) zum Überwachen der Gehirnaktivität eines Benutzers;
eine Steuerung (50), angepasst zum:

Bestimmen der Gehirnaktivität des Benutzers während einer Kalibrierung, gemessen durch die Sensoreinheit (40) als Reaktion auf einen Kalibrierungston, der durch die Tonerzeugungs-Einheit (30) erzeugt wird; und
während des Gebrauchs des Geräts (10), Einstellen der Signaleigenschaften des Maskierungstons, basierend auf:

Dem von der Wandlereinheit (20) erfassten Geräusch;
der Gehirnaktivität des Benutzers als Reaktion auf das Geräusch, das die Wandlereinheit (20) erfasst hat; und
der Gehirnaktivität des Benutzers als Reaktion auf den Kalibrierungston, der durch die Tonerzeugungseinheit (30) während der Kalibrierung erzeugt wurde;

Eine Speichereinheit, die mit der Steuerung (50) in Verbindung steht, angepasst, um Geräuschdaten basierend auf dem von der Wandlereinheit (20) erfassten Geräusch zu speichern; und **dadurch gekennzeichnet, dass** das Steuergerät (50) ferner angepasst ist zum:

Analysieren der Geräuschdaten unter Verwendung eines Algorithmus, wobei der Algorithmus vorzugsweise ein maschineller Lernalgorithmus ist;
eine erwartete Geräuschentwicklung zu bestimmen; und
die Signaleigenschaften des Maskierungstons basierend auf der erwarteten Geräuschentwicklung anzupassen.

2. Vorrichtung (10) nach Anspruch 1, wobei die Signaleigenschaften der maskierenden Geräusche mindestens eines der folgenden umfassen:

Eine Lautstärke des Signals; und
eine Frequenz des Signals.

3. Vorrichtung nach Anspruch 2, wobei:

Die Tonerzeugungseinheit (30) angepasst ist, um während der Kalibrierung einen Kalibrierungston erzeugen; und
die Steuerung (50) angepasst ist zum:

Bestimmen der Gehirnaktivität des Benutzers als Reaktion auf den Kalibrierungston;
Festlegen einer oberen und unteren Lautstärkegrenze für den Maskierungston, basierend auf der Gehirnaktivität des Benutzers als Reaktion auf den Kalibrierungston.

4. Vorrichtung (10) nach einem der Ansprüche 1 bis 3, wobei die Sensoreinheit (40) ein Elektroenzephalographie-(EEG)-System umfasst, sodass die Gehirnaktivität des Benutzers, die von der Sensoreinheit (40) gemessen wird, eine Elektroenzephalographie-Antwort umfasst.

5. Vorrichtung nach einem der Ansprüche 1 bis 4, die ferner eine Benutzeroberfläche umfasst, die mit der Steuerung (50) in Verbindung steht, angepasst, um es dem Benutzer (12) zu ermöglichen, die Lautstärke des Maskierungstons manuell einzustellen.

6. Vorrichtung nach Anspruch 5, wobei:

   Die Tonerzeugungseinheit (30) angepasst ist, um während der Kalibrierung einen Kalibrierungston erzeugen;
   Die Benutzeroberfläche angepasst ist, um Benutzerrückmeldung zum Kalibrierungston zu empfangen;
   und die Steuerung (50) angepasst ist, um eine obere Lautstärkegrenze und eine untere Lautstärkegrenze für den Maskierungston einzustellen, basierend auf der Benutzerrückmeldung.

7. Vorrichtung nach einem der Ansprüche 1 bis 6, wobei:

   Die Sensoreinheit (40) weiterhin angepasst ist, den Schlafzustand eines Benutzers zu erkennen;
   und die Steuerung (50) weiterhin angepasst ist, um die Signaleigenschaften des Maskierungstons basierend auf dem Schlafzustand des Benutzers anzupassen.

8. Vorrichtung nach Anspruch 7, wobei:
   Die Steuerung (50) weiterhin angepasst ist zum Bestimmen einer empfohlenen Anpassung der Eigenschaften des Maskierungstons, basierend auf dem Schlafzustand des Benutzers; und die Benutzeroberfläche ist weiterhin angepasst, um den Benutzer (12) zur empfohlenen Einstellung zu benachrichtigen.

9. Vorrichtung nach einem der Ansprüche 1 bis 8, wobei die Sensoreinheit (40) sich in drahtloser Kommunikation mit der Steuerung (50) befindet.

10. Verfahren zum Maskieren von Geräusch, wobei das Verfahren umfasst:

    (70,71,72,73; 80,84,85) während einer Kalibrierung,
    wobei die Gehirnaktivität des Benutzers bestimmt wird als Reaktion auf einen Kalibrierungston;
    (90) Erfassen eines zu maskierenden Geräusches;
    (92) Überwachung der Gehirnaktivität eines Benutzers; und (94) Erzeugen eines Maskierungstons und Anpassen der Signaleigenschaften des Maskierungstons, basierend auf dem erkannten Geräusch, der Gehirnaktivität des Benutzers als Reaktion auf das Geräusch, und der Gehirnaktivität des Benutzers als Reaktion auf den Kalibrierungston.
    Speichern von Geräuschdaten basierend auf dem erfassten zu maskierenden Geräusch;
    Analysieren der Geräuschdaten unter Verwendung eines Algorithmus, wobei der Algorithmus vorzugsweise ein maschineller Lernalgorithmus ist; eine erwarteten Geräuschentwicklung zu bestimmen; und die Signaleigenschaften des Maskierungstons basierend auf der erwarteten Geräuschentwicklung anzupassen.

11. Verfahren nach Anspruch 10, wobei das Kalibrierungsverfahren umfasst:

    (70) einen Kalibrierungston zu erzeugen; und
    (71) Überwachung der Gehirnaktivität des Benutzers als Reaktion auf den Kalibrierungston,
    und wobei die Anpassung der Signaleigenschaften des Maskierungstons umfasst:
    (72, 73) Eine obere und untere Lautstärkegrenze für den Maskierungston festzulegen, basierend auf der Gehirnaktivität des Benutzers als Reaktion auf den Kalibrierungston.

12. Verfahren wie beansprucht in einem der Ansprüche 10 oder 11, wobei der Schritt zum Bestimmen der Gehirnaktivität des Benutzers als Reaktion auf einen Kalibrierungston umfasst:

    (80) Erzeugen eines Kalibrierungstons;
    (84) Empfangen von Benutzer-Feedback zum Kalibrierungston; und
    (85) Festlegen einer oberen und unteren Lautstärkegrenze für den Maskierungston, basierend auf der Rückmeldung des Benutzers.

13. Ein Computerprogrammprodukt, das Anweisungen enthält, die, wenn das Programm auf einem Computer ausgeführt wird, den Computer veranlassen, das Verfahren von einem der Ansprüche 10 bis 12 auszuführen.

**Revendications**

1. Un dispositif (10) pour masquer le bruit, le dispositif (10) comprend:

   une unité de transducteur (20) pour détecter le bruit devant être masqué; une unité génératrice de son (30) pour générer un son masquant; une unité de détection (40) pour surveiller l'activité cérébrale d'un utilisateur; un contrôleur (50) adapté pour:

   lors d'un calibrage, déterminer l'activité cérébrale d'un utilisateur, mesurée par l'unité de détection (40), en réponse à un son calibré, généré par l'unité génératrice de son (30); et lors de l'utilisation du dispositif (10), ajuster les caractéristiques du signal du son masquant fondé sur:

   le bruit détecté par l'unité de transducteur (20);
   l'activité cérébrale de l'utilisateur en réponse au bruit détecté par l'unité de transducteur (20); et l'activité cérébrale de l'utilisateur en réponse à son de calibrage généré par l'unité de production de son (30) lors du calibrage;

   une unité de stockage de la mémoire en communication avec le contrôleur (50), adaptée pour stocker des données sur le bruit fondées sur le bruit détecté par l'unité de transducteur (20); et **caractérisé par le fait que** le contrôleur (50) est également adapté pour:

   analyser les données sur le bruit utilisant un algorithme, de préférence où l'algorithme est un algorithme d'apprentissage automatique;
   déterminer une tendance prévue en matière de bruit; et ajuster les caractéristiques de signal du son masquant fondé sur la tendance prévue en matière de bruit.

2. Un dispositif (10) comme revendiqué selon la revendication 1, où les caractéristiques du signal du son masquant comprend au moins:
   un volume de signal; et une fréquence de signal.

3. Un dispositif comme revendiqué dans la revendication 2, où:
   l'unité de production de son (30) est adaptée, lors du calibrage, pour générer un son de calibrage; et le contrôleur (50) est adapté pour:
   déterminer l'activité cérébrale d'un utilisateur en réponse au son de calibrage; établir une limite supérieure et inférieure de volume pour le son masquant fondé sur l'activité cérébrale de l'utilisateur en réponse au son de calibrage.

4. Un dispositif (10) comme revendiqué selon l'une quelconque des revendications 1 à 3, où l'unité de détection (40) comprend un système d'électroencéphalographie (EEG), de manière que l'activité cérébrale mesurée par l'unité de détection (40) comprend une réponse électroencéphalographique.

5. Un dispositif comme revendiqué selon l'une quelconque des revendications 1 à 4, comprend également une unité d'interface utilisateur, en communication avec le contrôleur (50), adapté pour permettre à l'utilisateur (12) pour ajuster manuellement le volume du son masquant.

6. Un dispositif comme revendiqué dans la revendication 5, où:

   l'unité de production du son (30) est adaptée, lors du calibrage, pour générer un son de calibrage;
   l'unité interface utilisateur est adaptée pour recevoir des commentaires utilisateur sur le son de calibrage; et le contrôleur (50) est adapté pour établir une limite supérieure du volume et une limite inférieure du volume pour le son masquant fondé sur les commentaires utilisateur.

7. Un dispositif comme revendiqué selon l'une des revendications 1 à 6, où:
   l'unité de détection (40) est également adaptée pour détecter un état de sommeil de l'utilisateur; et le contrôleur (50) est également adapté pour ajuster les caractéristiques du signal du son masquant fondé sur l'état de sommeil de l'utilisateur.

**8.** Un dispositif comme revendiqué dans la revendication 7, où:
le contrôleur (50) est également adapté pour déterminer un ajustement recommandé des caractéristiques du signal du son masquant fondé sur l'état de sommeil de l'utilisateur; et l'unité interface utilisateur est également adaptée pour informer l'utilisateur (12) de l'ajustement recommandé.

**9.** Un dispositif comme revendiqué selon l'une quelconque des revendications 1 à 8, où l'unité de détection (40) est en communication sans fil avec le contrôleur (50).

**10.** Une méthode pour masquer le son, la méthode comprend:

(70,71,72,73; 80,84,85) lors d'un calibrage, la détermination de l'activité cérébrale de l'utilisateur en réponse à son de calibrage;
(90) la détection d'un bruit devant être masqué;
(92) la surveillance de l'activité cérébrale de l'utilisateur; et
(94) générer un son masquant, et ajustant les caractéristiques du signal du son masquant fondé sur le bruit détecté, l'activité cérébrale de l'utilisateur en réponse au bruit, et l'activité cérébrale de l'utilisateur en réponse au son de calibrage. Stocker des données sonores fondées sur le bruit détecté devant être masqué; analyser les données sonores utilisant un algorithme, de préférence où l'algorithme est un algorithme d'apprentissage automatique;
déterminer une tendance prévue en matière de bruit; et ajuster les caractéristiques du signal du son masquant fondé sur la tendance prévue en matière de bruit.

**11.** Une méthode comme revendiquée dans la revendication 10, où la méthode de calibrage comprend:

(70) la production d'un son de calibrage;
et (71) surveiller l'activité cérébrale de l'utilisateur en réponse au son de calibrage, et où l'ajustement des caractéristiques du signal du son masquant comprend:
(72, 73) établir les limites supérieures et inférieures du volume pour le son masquant fondé sur l'activité cérébrale de l'utilisateur en réponse au son de calibrage.

**12.** Une méthode comme revendiquée selon l'une quelconque des revendications 10 ou 11, où l'étape de déterminer l'activité cérébrale de l'utilisateur en réponse à son de calibrage comprend:

(80) la production d'un son de calibrage;
(84) la réception des commentaires des utilisateurs sur le son de calibrage;
et (85) établir les limites inférieures et supérieures du volume pour le son masquant fondé sur les commentaires des utilisateurs.

**13.** Un produit de programme informatique comprend des instructions qui, lorsque le programme est exécuté par un ordinateur, fait en sorte que l'ordinateur exécute la méthode selon l'une quelconque des revendications 10 à 12.

FIG. 1

FIG. 2

70

71

72          73

## FIG. 3

80

84

85

## FIG. 4

90

92

94

## FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1886707 A
- WO 2018053114 A
- WO 2018166625 A

**Non-patent literature cited in the description**

- **ROESSLER, R. ; COLLINS, F. ; BURCH, N. R.** HEART RATE RESPONSE TO SOUND AND LIGHT. *Psychophysiology,* 1969, vol. 5, 359-369